# EUROPÄISCHE PATENTSCHRIFT

(11) **EP 1 651 569 B1**
(45) Veröffentlichungstag und Bekanntmachung des Hinweises auf die Patenterteilung: **18.06.2008**
(21) Anmeldenummer: 04763914.1
(22) Anmeldetag: 09.08.2004
(51) Int. Cl.: C02F 1/36, C02F 1/32

(54) **VERFAHREN ZUR DESINFEKTION VON FLÜSSIGKEITEN**
METHOD FOR DISINFECTING LIQUIDS
PROCEDE DE DESINFECTION DE LIQUIDES

(30) Priorität: 08.08.2003 DE 10336774; 03.11.2003 DE 10351184; 28.06.2004 DE 102004031273
(43) Veröffentlichungstag der Anmeldung: 03.05.2006
(73) Patentinhaber: Büttner, Klaus, D-25336 Klein Nordende (DE)
(72) Erfinder: Büttner, Klaus, D-25336 Klein Nordende (DE)
(74) Vertreter: Glaeser, Joachim
(86) Internationale Anmeldenummer: PCT/EP2004/008887
(87) Internationale Veröffentlichungsnummer: WO 2005/014489

(56) Entgegenhaltungen:
- EP-A- 1 008 556
- WO-A-96/20017
- WO-A-02/079096
- US-B1- 6 555 011

## Beschreibung

Die Erfindung bezieht sich auf ein Verfahren zur Behandlung von wässrigen Flüssigkeiten, nämlich Dialyseflüssigkeiten sowie Flüssigkeiten, die bei höchsten Anforderungen an ihre Reinheit praktisch frei von störenden Restbestandteilen sein müssen, zur Verringerung des Anteils an solchen Bestandteilen, die im Körper von Lebewesen unerwünscht oder gar gefährlich sind (vor allem Toxine).

Unter derartigen Bestandteilen werden in dieser Anmeldung u. a. Keime, Sporen, Herbizide, Fungizide, Pestizide, Restdüngemittel, Restmedikamente, Toxine und insbesondere Endotoxine verstanden.

Es ist bekannt Ultraschall einzusetzen, um Flüssigkeiten zu desinfizieren. Wenngleich hier Einflüsse auch auf Endotoxine zu erwarten sind, so hat sich doch in Versuchen gezeigt, dass mit Hilfe des Ultraschalls sich in biologischen Flüssigkeiten der Anteil an Endotoxinen nicht hinreichend herabsetzen lässt, schon gar nicht so, dass von Endotoxinfreiheit gesprochen werden kann, d.h. unter 0,4 EU/ml an Endotoxinen in der Flüssigkeit verbleiben.

Es ist auch bekannt, Wasserstoffperoxid zur Entkeimung von Flüssigkeiten einzusetzen, wenngleich dessen Reaktion mit organischen Substraten träge abläuft. Eine Einwirkung auf Endotoxine wäre nur dann möglich, wenn mit relativ hohen Konzentrationen vorgegangen wird. Dies führt dann zwangsläufig dazu, dass in der behandelten Flüssigkeit Rest-Wasserstoffperoxid verbleibt, das seinerseits die Zellwände in menschlichen Körper angreift, so dass der Versuch, auf diesem Wege die Endotoxineinwirkung zu verringern, unweigerlich zu einem nicht tragbaren Risiko führt.

Es ist auch bekannt, Keime in Flüssigkeiten mit Hilfe von UV-Strahlung zu inaktivieren. Versuche in dieser Richtung haben jedoch keine Verringerung des Anteils an Endotoxin in Flüssigkeiten gezeigt.

Die Behandlung von organischen Halogenzusammensetzungen, so dass diese als Abwasserflüssigkeiten abgegeben werden können, ist bekannt (WO-A-020 79 096, vgl. Übersetzung EP-A-1 375 432), wobei auch bemerkt wird, dass organische Substanzen und Stickstoff und Phosphor enthaltende zusammensetzungen zersetzt werden sollen. Bei den zu behandelnden Flüssigkeiten geht es um Wasser in Flüssen, Seen und Marschen. Die zugehörige Vorrichtung ist mit einem Reaktor ausgestattet, in dem die zu behandelnde Flüssigkeit mit Ultraschallwellen und Ultraviolettstrahlen behandelt wird, wobei die UV-Strahlungseinrichtung örtlich so angeordnet ist, dass diese mit dem Ausbreitungsweg der US-Wellen nicht interferiert bzw. die US-Wellen an der Oberfläche der UV-Strahlungseinrichtung entlang gehen.

Die Wirkung der bekannten Vorrichtung beruht auf der thermischen Zersetzung der zu behandelnden Flüssigkeit durch US-Wellen sowie auf der oxidativen Zersetzung durch OH-Radikale.

Bei einer bekannten Vorrichtung (WO 03/035 145 A1) werden Flüssigkeiten durch den Einsatz von OH-Radikalen und H₂O₂ sterilisiert und von Schadstoffen befreit. Hierzu wird die Flüssigkeit in Form einer chaotischen Strömungssäule oder als ein Oberflächenfilm mit geringer Dicke an der Oberfläche eines UV-Strahlers entlang geführt. Die OH⁻-Radikal-Erzeugung wird durch die Kombination der gewählten UV-Wellenlängen und/oder einem integrierten Rührwerk, vorzugsweise einem Ultraschallgenerator, eingeleitet und aufrecht erhalten. Beim Einsatz zur Behandlung von sog. Dialyseflüssigkeiten werden daher Sterilfilter erforderlich.

Der Erfindung liegt die Aufgabe zu Grunde, das Verfahren der eingangs genannten Art so auszugestalten, dass Flüssigkeiten von störenden Bestandteilen befreit werden können, was durch die nachfolgend noch im einzelnen erläuterten Verfahrensslufen A, B und C erreicht wird.

Unter Toxinen oder Endotoxinen sollen allgemein Giftstoffe von Mikroorganismen verstanden werden, die von den äußeren Zellmembranen gramnegativer Bakterien stammen und im Wesentlichen aus thermostabilen Lipopolysacchariden bestehen. Die toxische Komponente der Endotoxine ist das Lipid A, ein Phospholipid aus einem Glucosamindisaccharid, das an den Hydroxyl - und Aminogruppen mit Fettsäuren verestert ist. Größere Mengen von Endotoxinen werden beim Absterben von gramnegativen Bakterien frei und können zu Schockerscheinungen bei den betroffenen Personen führen.

Die vorliegende Erfindung soll jedoch nicht auf die Behandlung von Flüssigkeiten hinsichtlich der Endotoxinfreiheit begrenzt sein, sondern auch allgemein einsetzbar sein, wenn es darum geht, derartige Bestandteile aus wasserhaltigen Flüssigkeiten zu entfernen.

Für die vorliegende Erfindung ist nicht nur die Kombination von Verfahrensmerkmalen wesentlich, bedeutsam sind auch die sich aus dem Einsatz dieser Merkmale sich ergebenden Wechselwirkungen, die bislang in diesem Zusammenhang nicht beschrieben worden sind.
A. Einwirken auf die Flüssigkeit mittels mindestens einer sich innerhalb der Flüssigkeit befindlichen Ultraschallquelle, durch die über Kavitationsvorgänge in der Flüssigkeit Hydroxylradikale aus dem wässrigen Anteil der Flüssigkeit gebildet werden, wobei die zur Ultraschallquelle gehörenden US-Strahler in Strömungsrichtung der Flüssigkeit mit Frequenzen von ca 20 bis 400, sodann 400 bis 800 und abschließend 800 bis 3000 kHz auf die Flüssigkeit einwirken,
B. Aufspalten der Bestandteile bzw. Endotoxine durch die Hydroxylradikale in unwirksame Restbestandteile bzw. Endotoxinreste, wobei auch Wasserstoffperoxid durch die Hydroxylradikale entsteht, und
C. Einwirken auf das Wasserstoffperoxid, mittels einer UV-Strahlung zur Bildung weiterer Hydroxylradikale aus dem Wasserstoffperoxid, die ihrerseits wiederum zur zusätzlichen Aufspaltung von Bestandteilen bzw. Endotoxinen (gemäß Stufe B) führen.

Bei einer weiteren Ausführungsform geht es um ein Verfahren zur Behandlung eines Fluids oder einer wässrigen Lösung in einem Reaktionsraum, **gekennzeichnet durch**
Einleiten eines H₂O₂-Aerosols und/oder eines Peressigsäure-Aerosols in den Reaktionsraum, und durch
Einwirken auf das Fluid durch ein Aerosol aus Hydroxylradikalen, die aus dem H₂O₂-Aerosol und/oder dem Peressigsäure-Aerosol durch UV-Strahler im Reaktionsraum gebildet werden,

Bei einer weiteren Ausführungsform geht es um ein Verfahren, bei dem auf die Flüssigkeit bzw. das Fluid und das Hydroxylradikal-Aerosol durch einen Schallgenerator oder Ultraschallgenerator im Reaktionsraum eingewirkt wird, um eine gute Durchmischung bzw. Verwirbelung und eine Oberflächenvergrößerung zu erzielen.

Bei einer weiteren Ausführungsform geht es um ein Verfahren, bei dem in einem separaten Reaktionsraum mittels eines Ultraschallgenerators H₂O₂- und/oder Peressigsäure-Aerosole gebildet werden.

Bei einer weiteren Ausführungsform geht es um ein Verfahren, bei dem das Einwirken auf das Wasserstoffperoxid mittels einer UV-Strahlung mit einer Wellenlänge von vorzugsweise 253,7 nm erfolgt.

Bei einer weiteren Ausführungsform geht es um ein Verfahren, bei dem das Einwirken auf die Flüssigkeit in einem Druckreaktionsraum unter erhöhtem Druck bis zu 4000 bar erfolgt.

Es geht bei der Erfindung zunächst einmal darum, Kavitationserscheinungen in einer Flüssigkeit durch die Einwirkung einer Ultraschallquelle zu erzeugen. Kavitationserscheinungen werden in strömenden Flüssigkeiten bei Geschwindigkeitsänderungen beobachtet, wobei bei Beschleunigung sich Druckerniedrigungen ergeben können, die zu Dampfbläschen führen, wobei sich Druckstöße von ca. 10.000 bar und örtliche Temperaturerhöhungen im Bereich von ca. 10.000 K ergeben können, die sich an benachbarten Körpern, also z.B. an Laufrädern von Wasserturbinen oder Schiffsschrauben zerstörerisch bemerkbar machen.

Bei der vorliegenden Erfindung geht es jedoch um Kavitationserscheinungen in der Flüssigkeit selbst und daher ist ein wesentliches Merkmal, dass die Ultraschallquelle, die Verursacher der Kavitationserscheinungen ist, sich innerhalb der zu behandelnden Flüssigkeit befindet. Dadurch bilden sich in der Nähe der Ultraschallquelle vor allem Hydroxylradikale, was bei der Erfindung im Einsatz gegen Endotoxine erwünscht ist. Die Hydroxylradikale wirken auf die Endotoxine ein und es ergeben sich Vorgänge, bei denen sich Wasser, Kohlendioxid und unwirksame Endotoxinreste ergeben.

Kavitation und UV-Strahlung wirken in einer Art energetischer Koppelung zumindest in einem gewissen Raum und zur gleichen Zeit in der zu behandelnden Flüssigkeit miteinander. Die sich im Einzelnen abspielenden physikalischen Vorgänge sind noch nicht hinlänglich untersucht worden. Es hat sich jedoch gezeigt, dass der mit der Erfindung erzielte Wirkungsgrad (z. B. Endotoxinfreiheit) deutlich erhöht werden kann, wenn der Druck im Reaktor erhöht wird.

Nun haben Versuche gezeigt, dass nicht alle Hydroxylradikale auf Endotoxine treffen, sondern eben durchaus auch mit anderen Hydroxylradikalen reagieren können und hierdurch entsteht Wasserstoffperoxid.

Es ist nun weiterhin erkannt worden, dass Wasserstoffperoxid bei der Behandlung von biologischen Flüssigkeiten durchaus nicht erwünscht ist, sondern dass eine Vorkehrung getroffen werden muss, um die Wasserstoffperoxidmoleküle in ihrer Wirkung auszuschalten oder besser noch im Sinne der Erfindung förderlich einzusetzen. Dies wird durch den zusätzlichen Einsatz einer UV-Quelle möglich gemacht, die nun wiederum bewirkt, dass Wasserstoffperoxidmoleküle in erwünschte Hydroxylradikale umgebildet werden, die in der bereits beschriebenen Art und Weise auf Endotoxine einwirken.

Mit Hilfe des Verfahrens gemäß der Erfindung konnten Flüssigkeiten nicht nur im Sinne der Definition endotoxinfrei gemacht werden, sondern so weit von wirksamen Endotoxinen befreit werden, dass der Gehalt an verbliebenen Endotoxinen unterhalb der Nachweisgrenze (0,06 EU/ml) lag.

Nachfolgend einige ergänzende Erläuterungen zu den Merkmalen der Erfindung:
Einwirken auf die Flüssigkeit mittels einer solchen innerhalb der Flüssigkeit befindlichen Ultraschallquelle, durch die über Kavitationsvorgänge in der Flüssigkeit Hydroxylradikale aus dem wässrigen Anteil der Flüssigkeit gebildet werden.

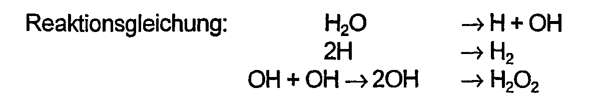

Der physikalisch chemische Prozess läuft in reinem Wasser so ab. Wenn aber im Wasser biologische Verunreinigungen - z. B. Endotoxine - sind, kann der physikalisch chemische Prozess auch so ablaufen:

OH+Endotoxin → H₂O+CO₂ + N...-(Stickstoffrest) = Entwicklungsziel: unwirksames Endotoxin.

Aufspalten der Endotoxine durch die Hydroxylradikale in unwirksame Endotoxinreste, wobei auch Wasserstoffperoxid durch die Hydroxyl-radikale entsteht. Ein Hydroxylradikal hat eine Halbwertzeit von 1 ns (10⁻⁹ sec), also eine Zeit, sich zu orientieren und reagiert wie folgt:

OH + OH → H₂O₂

oder mit einem Endotoxin

OH+Endotoxin → H₂O+CO₂ + N...-(Stickstoffrest) = Enttwicklungsziel: unwirksames Endotoxin

Einwirken auf die Wasserstoffperoxydmoleküle mittels einer UV-Strahlung zur Bildung weiterer Hydroxylradikale aus dem Wasserstoffperoxyd, die ihrerseits wiederum zur zusätzlichen Aufspaltung von Endotoxinen (gemäß Stufe B) führen.

Weil die jeweilige "Lebenszeit" für ein Hydroxylradikal von einer Nanosekunde zu kurz ist und die Menge der erzeugten Hydroxylradikale aus verständlichen und vor allen Dingen **notwendigen** Gründen gering sein muss, wird mit den UV-Strahlen ein Wirkmechanismus eingesetzt, die **geringe** Menge der erzeugten Hydroxylradikale solange wirksam zu behalten, bis sie ein Endotoxin "gefunden" haben.

Reaktionsgleichung: H₂O₂ + *hv*_{(UV)} → OH + OH

### Schlussfolgerung:

So wenig Hydroxylradikale wie möglich - aber so viel wie nötig - durch Schall / Kavitation erzeugen. Damit eine gute Ausbeute erzielt wird, wird durch UV die Bildung von H₂O₂ dauernd unterbrochen, so dass die aggressiven Hydroxylradikale irgendwann (Verweildauer oder Flüssigkeit im Reaktorfeld, das ist der Bereich, wo Kavitation und UV gleichzeitig wirken!), an die Endotoxine gelangen, es also einen Treffer gibt. Im Idealfall werden die Hydroxylradikale auf diese Weise "verbraucht".

Hierzu sind folgende Kriterien wichtig:

| | |
|---|---|
| **Für Schall:** | Schallfrequenz, Energieeintrag durch Schall und Verweildauer der Flüssigkeit im Reaktor. Das "Rühren" im molekularen Bereich durch Schall und die durch Schall erzeugte Rotation der Moleküle. |
| **Für UV:** | Wahl der Frequenz, beispielsweise mit einer Wellenlänge von 253,7 nm, keimtötend und Aufbrechen der H₂O₂ -Bindungen. |

Nachfolgend aufgezählte Vorteile sind auf die Druckerhöhung der zu beschallenden Flüssigkeit maßgeblich zurückzuführen:

In der zu behandelnden Flüssigkeit gegebenenfalls vorhandene Tenside schäumen nicht auf und können so eliminiert werden, weil in Schaumblasen keine Kavitationsvorgänge stattfinden.

Die Schallgeneratoren laufen nicht trocken und können nicht zerstört werden, was bei Vorhandensein von Blasen auftreten könnte.

Der sog. JET aus der Kavitationsblase wird verstärkt, weil er gegen eine komprimierte ("härtere") Wasserfront trifft.

Wenn zufolge der JET-Verstärkung eine geringere Schallenergie ausreicht, den gleichen Kavitations-Effekt zu erreichen, ergibt sich daraus die Schonung der Schallmembran, geringerer Kavitationsfraß und eine Erhöhung der Standzeit des Schallgenerators.

Auch findet eine Dämpfung der Schallmembran statt, so dass es zu keinen Überlagerungsschwingungen kommt.

Bei der bekannten Keimreduzierung durch UV-Behandlung von Wasser wird in der Regel nur die DNA der Mikroorganismen geschädigt. Die dadurch möglichen Mutationen können zu neuen Stämmen mit unbekannten Merkmalen führen, so dass Resistenzbildung zu befürchten ist. Zudem absorbieren Schwebstoffe UV-Strahlen stärker als Ultraschall. Ultraschall zertrümmert Schwebstoffe. Vorgänge, die bei der Erfindung nicht zu befürchten sind.

Die Erfindung wird nachstehend anhand der Zeichnung beispielsweise erläutert.

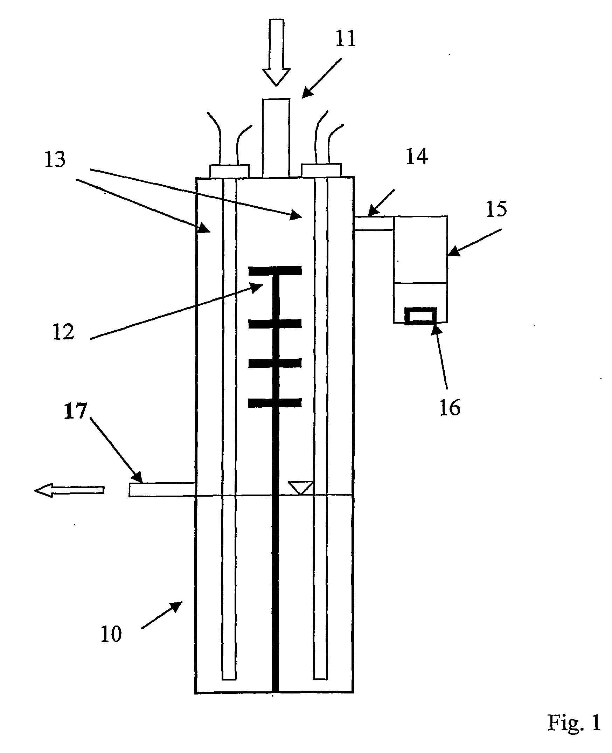

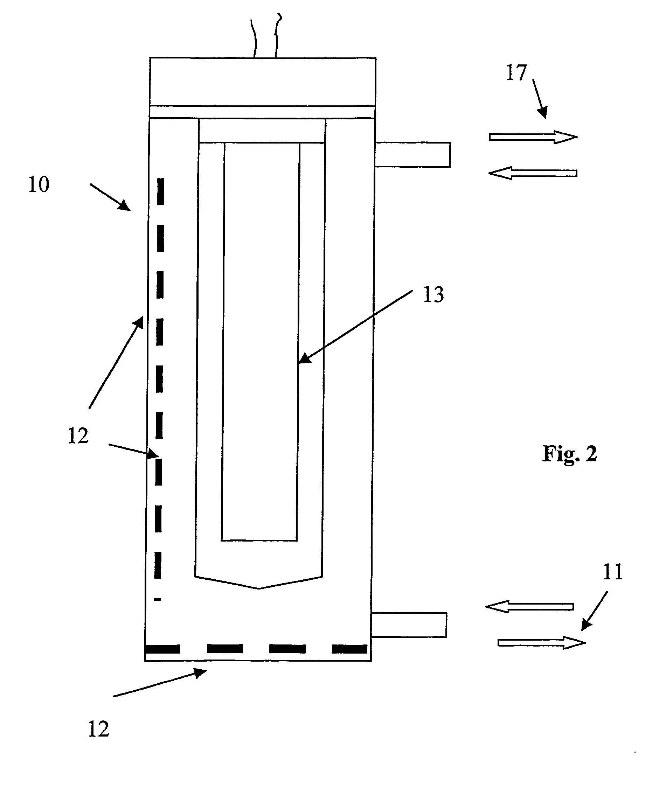

Die Figuren 1 und 2 zeigen schematische Schnittansichten durch Vorrichtungen, die geeignet sind, Verfahren gemäß der Erfindung durchzuführen.

Im Mittelteil der Figuren ist ein länglicher Behälter 10 gezeigt, der beispielsweise Zylinderform haben kann und als Druckreaktionsraum dient und unter erhöhtem Druck steht.

Gemäß Fig. 1 ist im oberen Bereich ein mittlerer Einlass 11 für die zu behandelnde Flüssigkeit vorgesehen. Die eingeleitete Flüssigkeit gelangt zu mehreren Schallgeneratoren 12, die mit Frequenzen von 40 kHz bis ca. 3,0 MHz arbeiten, Hierdurch wird die Flüssigkeit fein zerstäubt und es entstehen Aerosol-Tröpfchen, die klein genug sind, dass sie von UV-Strahlen leicht durchdrungen werden können. Die UV-Strahlen stammen von UV-Strahlern 13, die von oben und außen her mit Energie versorgt werden und sich im Wesentlichen in Längsrichtung des Behälters 10 erstrecken. Bei einer Ausführungsform liegen die Frequenzbereiche (Angaben in KHz) in Strömungsrichtung der Flüssigkeit bzw. des Fluids zwischen ca. 20 bis 400, sodann 400 bis 800 und abschließend 800 bis 3000.

Im oberen Teil ist ein Zulauf 14 gezeigt, der zu einem separaten Reaktionsraum 15 führt. In diesem Reaktionsraum 15 befindet sich ein Ultraschallgenerator 16. In dem Reaktionsraum 15 werden Radikalbildner erzeugt. Dies wird dadurch bewerkstelligt, dass in dem Reaktionsraum 15 Wasserstoffperoxid eingeleitet wird und durch die Energie des Schallgenerators 16 die entsprechenden OH-Aerosole erzeugt werden. Die Aerosole gelangen über die Zuleitung 14 in den eigentlichen Reaktionsraum 10.

Im Reaktionsraum 10 wirken die Aerosol-Bildner auf die eingeleitete Flüssigkeit ein und die unerwünschten Bestandteile in der zu behandelnden Flüssigkeit, insbesondere Endotoxine, werden zu unwirksamen Resten umgewandelt.

Zunächst sammelt sich im unteren Teil des Reaktionsraumes 10 die so behandelte Flüssigkeit. Der Spiegel steigt sodann soweit an, bis der Überlauf 17 erreicht wird, so dass dort die behandelte Flüssigkeit austreten kann. Die sich im unteren Bereich des Gehäuses 10 sammelnde Flüssigkeit wird auch von UV-Brennern 13 erreicht, so dass auch hier Keimfreiheit sichergestellt werden kann.

In Fig. 2 werden für entsprechende Teile der Vorrichtung die Bezugszahlen der Fig. 1 verwendet. Jedoch ist dort der Einlass 11 unten und der Auslass 17 oben. Die umgekehrte Strömungsrichtung wird durch die Pfeile angedeutet.

Von besonderer Bedeutung ist die Anordnung der Schallgeneratoren, nämlich außen und unten im Innenraum des Behälters 10.

## Patentansprüche

1. Verfahren zur Behandlung von wässrigen Flüssigkeiten, nämlich Dialyseflüssigkeiten sowie Flüssigkeiten, die bei höchsten Anforderungen an ihre Reinheit praktisch frei von störenden Restbestandteilen sein müssen, zur Verringerung des Anteils an solchen Bestandteilen, die im Körper von Lebewesen unerwünscht oder gar gefährlich sind (vor allem Toxine), umfassend
A. Einwirken auf die Flüssigkeit mittels mindestens einer sich innerhalb der Flüssigkeit befindlichen Ultraschallquelle, durch die über Kavitationsvorgänge in der Flüssigkeit, Hydroxylradikale aus dem wässrigen Anteil der Flüssigkeit gebildet werden, wobei die zur Ultraschallquelle gehörenden US-Strahler in Strömungsrichtung der Flüssigkeit mit Frequenzen von ca. 20 bis 400, sodann 400 bis 800 und abschließend 800 bis 3000 kHz auf die Flüssigkeit einwirken,
B. Aufspalten der Bestandteile bzw. Endotoxine durch die Hydroxylradikale in unwirksame Restbestandteile bzw. Endotoxinreste, wobei auch Wasserstoffperoxid durch die Hydroxylradikale entsteht, und
C. Einwirken auf das Wasserstoffperoxid mittels einer UV-Strahlung zur Bildung weiterer Hydroxylradikale aus dem Wasserstoffperoxid, die ihrerseits wiederum zur zusätzlichen Aufspaltung von Bestandteilen bzw. Endotoxinen (gemäß Stufe B) führen.

2. Verfahren nach Anspruch 1 zur Behandlung eines Fluids oder einer wässrigen Lösung in einem Reaktionsraum, **gekennzeichnet durch**
Einleiten eines H₂O₂-Aerosols und/oder eines Peressigsäure-Aerosols in den Reaktionsraum, und **durch**
Einwirken auf das Fluid **durch** ein Aerosol aus Hydroxylradikalen, die aus dem H₂O₂-Aerosol und/oder dem Peressigsäure-Aerosol **durch** UV-Strahler in Reaktionsraum gebildet werden,

3. Verfahren nach einem der Ansprüche 1 oder 2, **dadurch gekennzeichnet, dass** auf die Flüssigkeit bzw. das Fluid durch einen Schallgenerator oder Ultraschallgenerator im Reaktionsraum eingewirkt wird, um eine gute Durchmischung bzw. Verwirbelung und eine Oberflächenvergrößerung zu erzielen.

4. Verfahren nach mindestens einem der Ansprüche 1 bis 3, **dadurch gekennzeichnet, dass** in einem separaten Reaktionsraum mittels eines Ultraschallgenerators H₂O₂- und/oder Peressigsäure-Aerosole gebildet werden.

5. Verfahren nach mindestens einem der Ansprüche 1 bis 4, **dadurch gekennzeichnet, dass** das Einwirken auf das Wasserstoffperoxid mittels einer UV-Strahlung mit einer Wellenlänge von vorzugsweise 253,7 nm erfolgt.

6. Verfahren nach Anspruch 5, **dadurch gekennzeichnet, dass** das Einwirken auf die Flüssigkeit in einem Druckreaktionsraum unter erhöhtem Druck bis zu 4000 bar erfolgt.

## Claims

1. Method for treating aqueous liquids, namely dialysis liquids as well as liquids, which have to be practically free of disturbing residual components under highest requirements regarding their purity, for reducing the proportion of such components, which are undesired in the body of creatures or are even dangerous (above all, toxins), comprising
A. Acting upon the liquid by means of at least one ultrasound source located in the liquid, whereby, through cavitation processes in the liquid, hydroxyl radicals are formed from the aqueous part of the liquid, wherein the ultrasound radiators included in the ultrasound source act upon the liquid in the flow direction of the liquid with frequencies of about 20 to 400, then 400 to 800 and finally 800 to 3000 kHz.
B. Decomposing the components or endotoxins, respectively, by the hydroxyl radicals into ineffective residual components or endotoxin residuals, wherein also hydrogen peroxide is generated by the hydroxyl radicals, and
C. Acting upon the hydrogen peroxide by means of ultraviolet radiation in order to form further hydroxyl radicals from the hydrogen peroxide, which in turn result in additional decomposition of components or endotoxins (according to step B).

2. Method according to claim 1 for treating a liquid or an aqueous solution in a reaction chamber, **characterised by**
introducing a H₂O₂ aerosol and/or a peracetic acid aerosol into the reaction chamber, and by
acting upon the fluid by means of an aerosol consisting of hydroxyl radicals, which are formed by the H₂O₂ aerosol and/or the peracetic acid aerosol by ultraviolet radiators in the reaction chamber.

3. Method according to one of the claims 1 or 2, **characterised in that** a sound generator or ultrasound generator acts upon the liquid or the fluid, respectively, in the reaction chamber, in order to achieve a good mixing or swirling and a surface extension.

4. Method according to at least one of the claims 1 to 3, **characterised in that** H₂O₂ and/or peracetic acid aerosols are formed in a separate reaction chamber by means of an ultrasound generator.

5. Method according to at least one of the claims 1 to 4, **characterised in that** the hydrogen peroxide is acted upon by means of ultraviolet radiation at a wavelength of preferably 253,7 nm.

6. Method according to claim 5, **characterised in that** the liquid is acted upon in a pressure reaction chamber under increased pressure of up to 4000 bar.

## Revendications

1. Procédé de traitement de liquides aqueux, plus particulièrement de liquides pour la dialyse et de liquides qui, pour répondre aux exigences de pureté les plus élevées, doivent être pratiquement exempts de composants résiduels, en vue de la diminution de la partie de tels composants qui sont indésirables dans le corps des êtres vivants ou qui peuvent même être dangereux (toxines), comprenant les étapes suivantes :
A. Action sur le liquide d'au moins une source d'ultrasons localisée dans le liquide, grâce à laquelle des radicaux hydroxyles sont formés à partir de la partie aqueuse du liquide au moyen de processus de cavitation dans le liquide, les émetteurs d'ultrasons appartenant à la source d'ultrasons agissant en direction de l'écoulement du liquide à des fréquences comprises entre 20 et 400 kHz, puis entre 400 et 800 kHz et finalement entre 800 et 3000 kHz,
B. Division des composants, respectivement des endotoxines, par les radicaux hydroxyles en composants résiduels, respectivement en endotoxines résiduelles, du peroxyde d'hydrogène se formant également du fait des radicaux hydroxyles, et,
C. Action sur le peroxyde d'hydrogène d'un rayonnement ultraviolet en vue de former d'autres radicaux hydroxyles à partir du peroxyde d'hydrogène, qui à leurs tours conduisent à une division complémentaire des composants, respectivement d'endotoxines (selon l'étape B).

2. Procédé selon la revendication 1 pour le traitement d'un fluide ou d'une solution aqueuse dans un espace de réaction, **caractérisé par** l'introduction dans l'espace de réaction d'un aérosol de H₂O₂ et/ou d'un aérosol d'acide peroxyacétique et par l'action sur le fluide d'un aérosol de radicaux hydroxyles qui sont produits dans l'espace de réaction par des rayonnements ultraviolets à partir de l'aérosol H₂O₂ et/ou de l'aérosol d'acide peroxyacétique.

3. Procédé selon l'une des revendications 1 ou 2 **caractérisé en ce que** un générateur de sons ou un générateur d'ultrasons agit dans l'espace de réaction sur le liquide, respectivement le fluide, afin d'obtenir un bon mélange, respectivement un bon tourbillonnement et un agrandissement de la surface.

4. Procédé selon au moins une des revendications 1 à 3 **caractérisé en ce que** dans un espace de réaction séparé, des aérosols H₂O₂ et/ou des aérosols d'acide peroxyacétique sont obtenus par un générateur d'ultrasons.

5. Procédé selon au moins une des revendications 1 à 4 **caractérisé en ce que** l'action du rayonnement ultraviolet sur l'acide peroxyacétique est effectuée de préférence à une longueur d'onde de 253,7 nm.

6. Procédé selon la revendication 5 **caractérisé en ce que** l'action sur le liquide est effectuée dans un espace de réaction sous pression à une pression plus élevée pouvant atteindre 4000 bars.
